# EUROPÄISCHE PATENTANMELDUNG

(11) **EP 0 634 191 A2**
(43) Veröffentlichungstag der Anmeldung: **18.01.1995**
(21) Anmeldenummer: 94109489.8
(22) Anmeldetag: 20.06.1994
(51) Int. Cl.: A61N 1/05

(54) **Implantierbarer Defibrillator**

(30) Priorität: 02.07.1993 DE 4322130
(71) Anmelder: Pacesetter AB, S-171 95 Solna (SE)
(72) Erfinder: Stroetmann, Brigitte, Dr., D-91080 Uttenreuth (DE); Starbeck, Gerhard, D-90411 Nürnberg (DE)
(74) Vertreter: Lettström, Richard Wilhelm

(57) **Zusammenfassung**

Bei einem implantierbaren Defibrillator mit einer Flächenelektrode in Form eines Netzes, einer Spirale oder eines Gewebes aus elektronisch leitendem Material bzw. mit einer intrakardialen Elektrode in Form einer Wendel aus elektronisch leitendem Material ist die Elektrode vollständig in ein biokompatibles, hydrophiles, elektrolytisch leitendes Polymer eingebettet bzw. von einem derartigen Polymer umhüllt.

## Beschreibung

Die Erfindung betrifft einen implantierbaren Defibrillator mit einer Flächenelektrode in Form eines Netzes, einer Spirale oder eines Gewebes aus elektronisch leitendem Material bzw. mit einer intrakardialen Elektrode in Form einer Wendel aus elektronisch leitendem Material sowie verfahren zur Herstellung eines derartigen Defibrillators.

Defibrillatoren haben die Aufgabe, abnorme Herzfrequenzen, d.h. Rhythmusstörungen und Arrhythmien, zu detektieren und zu behandeln (siehe beispielsweise EP-OS 0 475 027). Die Wahrnehmung der Rhythmusstörungen erfolgt beispielsweise über myokardiale Schraubelektroden. Diese stellen ein Kammerflattern oder -flimmern beispielsweise durch Messen der Herzfrequenz oder durch Aufnahme eines Elektrokardiogramms (EKG) fest. Beim Erkennen von Kammerflattern bzw. -flimmern wird nach wenigen Sekunden ein Elektroschock abgegeben. Dabei wird der Stromstoß dem Herzen über Flächenelektroden (Patches) und/oder intrakardiale Elektroden aufgeprägt (siehe beispielsweise DE-OS 39 14 662).

Als Wirkungsmechanismus der elektrischen Defibrillation wird eine synchrone Reizung aller nicht refraktären Myokardbezirke angenommen. Es ist deshalb erforderlich, daß der gesamte Myokard-Zellverband gleichzeitig gereizt wird. Dies setzt aber eine ausreichende Stromdichte voraus.

Die Elektroden von Defibrillatoren müssen flexibel sein, damit sie sich den physiologischen Gegebenheiten des Körpers und des Herzens anpassen können. Außerdem müssen sie biokompatibel sein und ihre Oberfläche soll das umgebende Gewebe nicht durch zusätzliche Reibung reizen. Korrosionsbeständigkeit, niedrige Polarisation und Langzeitbeständigkeit sind ferner wichtige Kriterien, welche derartige Elektroden erfüllen müssen.

Flächenelektroden für Defibrillatoren bestehen bislang aus Metallnetzen oder -spiralen, die auf einer Seite in eine Schicht aus Silicongummi eingebettet und auf der anderen Seite kontaktiert sind. Während die Netze im allgemeinen aus Titan bestehen, sind die Spiralen aus Platin. Zwischen dem elektrisch leitenden Netz bzw. der Spirale und dem umgebenden Gewebe besteht ein direkter Kontakt, der - aufgrund der Rauhigkeit des Metalls - zu Reibungen mit dem Gewebe führen kann. Intrakardiale Elektroden können aus nichtmetallischen, elektrisch leitenden Fasern bestehen.

Aufgabe der Erfindung ist es, implantierbare Defibrillatoren mit einer Flächenelektrode bzw. intrakardialen Elektrode der eingangs genannten Art hinsichtlich Tauglichkeit und Gebrauchsfähigkeit weiter zu verbessern.

Dies wird erfindungsgemäß dadurch erreicht, daß die Elektrode vollständig in ein biokompatibles, hydrophiles, elektrolytisch leitendes Polymer eingebettet bzw. von einem derartigen Polymer umhüllt ist.

Beim implantierbaren Defibrillator nach der Erfindung wird durch den speziellen Aufbau der Elektrode vermieden, daß der elektronische Leiter im direkten Kontakt mit dem Gewebe steht. Da außerdem das Polymer - aufgrund seines hydrophilen Charakters - beim Kontakt mit der Körperflüssigkeit leicht aufquillt, erzeugt der Übergang zwischen der leitenden Struktur und dem Körpergewebe lediglich eine geringe Reibung ("Polsterwirkung").

Im Gegensatz zum bislang verwendeten Silicon, das isolierend wirkt, wird beim Defibrillator nach der Erfindung ein elektrolytisch leitendes Polymer eingesetzt. Dies ist insbesondere dann von großem Vorteil, wenn beim Ausfall des (internen) Defibrillatorsystems eine externe Defibrillation erforderlich ist. Die externe Defibrillation kann in diesem Fall nämlich mit geringen Energiemengen erfolgen. Daraus ergibt sich der weitere Vorteil, daß das Gewebe nicht unnötig mit hoher Energie belastet wird.

Das Polymer ist im allgemeinen ein Ionenaustauscher. Neben Anionenaustauschern werden vorteilhaft Kationenaustauscher eingesetzt, vorzugsweise in Form von Poly(perfluoralkylen)sulfonsäuren, insbesondere sulfoniertem Polytetrafluorethylen. Dies hat den Vorteil, daß die Oberfläche der Elektrode freigehalten wird von negativ geladenen Makromolekülen. Dadurch gestaltet sich die Entfernung der Elektrode nach einer bestimmten Implantationsdauer einfacher. Ein weiteres Beispiel für einen geeigneten Kationenaustauscher ist ein sulfoniertes Copolymer aus Styrol und Divinylbenzol. Anstelle von Sulfonsäuregruppen können die Kationenaustauscher beispielsweise auch Carbonsäure- oder Phosphorsäuregruppen aufweisen.

Die Dicke der Flächenelektrode ist vorteilhaft ≦ 300 µm. Auf diese Weise wird eine hohe Flexibilität gewährleistet. Als Material für die Flächenelektrode und für die intrakardiale Elektrode dient vorzugsweise Platin oder Platin/Iridium; daneben kann als Elektrodenmaterial beispielsweise auch Titan verwendet werden. Bei Flächenelektroden kann das Elektrodenmaterial vorteilhaft auch leitfähiger Kohlenstoff sein. Dazu dienen insbesondere Gewebe aus leitfähigem Kohlenstoff, beispielsweise in Form eines Geflechtes aus Kohlenstoff-Faserbündeln.

Beim implantierbaren Defibrillator nach der Erfindung kann zur Herstellung der Elektrode in der Weise vorgegangen werden, daß ein elektronisch leitendes Netz oder eine entsprechende Spirale in geeigneter Weise mit einem Polymer umhüllt wird. Vorteilhaft wird jedoch ein Netz oder eine Spirale aus elektronisch leitendem Material zwischen zwei Membranen aus einem biokompatiblen, hydrophilen, elektrolytisch leitenden Polymer angeordnet und diese Anordnung bei erhöhter Temperatur und erhöhtem Druck gepreßt und anschließend das Netz bzw. die Spirale kontaktiert.

Durch eine derartige Vorgehensweise wird erreicht, daß das Polymer fest auf dem Netz bzw. auf der Spirale haftet, und gleichzeitig wird die erforderliche mechanische Stabilität gewährleistet. Außerdem sind die beiden Membranen - samt dem Netz bzw. der Spirale - derart miteinander verpreßt, daß sie sich während des Defibrillatorschocks, der mit einer starken Gasentwicklung verbunden ist, nicht voneinander lösen.

Gewebe aus leitfähigem Kohlenstoff und Wendein aus elektronisch leitendem Material können mit einer Polymerumhüllung versehen werden, beispielsweise in Form eines Schlauches. Die Umhüllung kann aber auch in der Weise erfolgen, daß die genannten Materialien mit einer Polymerlösung behandelt werden, beispielsweise in eine derartige Lösung getaucht werden.

Vorteilhaft kann in das Polymer ein entzündungshemmendes Steroid eingelagert sein. Dazu kann die mit dem Polymer versehene Elektrode mit einer Polymer und Steroid enthaltenden Suspension behandelt werden.

Die auf die beschriebene Weise hergestellte kontaktierte Elektrode wird mit einer geeigneten Elektronik kombiniert und ergibt so einen Defibrillator, der zur Implantation geeignet ist.

Anhand eines Ausführungsbeispiels soll die Erfindung noch näher erläutert werden.

Ein 0,06 mm dickes Platinnetz (Maschenweite: 0,25 mm) mit den Abmessungen 50 mm x 80 mm wird zwischen zwei je ca. 180 µm dicke handelsübliche Membranen aus sulfoniertem Polytetrafluorethylen in der F⁻-Form (Nafion^{R} 117) angeordnet. Diese Anordnung (Gesamtdicke: ca. 420 µm) wird zwischen zwei je 0,15 mm dicke getemperte Niobbleche gelegt, um beim Pressen ein Anhaften der Membranen an der Preßfläche zu verhindern. Zum Pressen wird die gesamte Anordnung in einen 0,3 mm dicken Metallrahmen eingebracht und bei einem Druck von ca. 30 bar und einer Temperatur von ca. 230°C 10 min lang gepreßt. Anschließend wird innerhalb von 8 min auf eine Temperatur von 25°C abgekühlt. Die aus der Preßanordnung entnommenen Elektroden weisen eine Dicke von ca. 300 µm auf.

Zur Überführung der F⁻-Form des Polymers, d.h. des Ionenaustauschermaterials, in die H⁺-Form wird eine Hydrolyse und Konditionierung durchgeführt, was in an sich bekannter Weise erfolgt. Dazu wird die Elektrode zunächst ca. 6 h mit einer Mischung aus einer 2,5-molaren Natronlauge und Ethanol (im Verhältnis 5:1) bei einer Temperatur von 65°C behandelt. Anschließend wird die Elektrode aus der Lösung genommen und mit Wasser neutral gewaschen. Nachfolgend wird die Elektrode 30 min in siedendes Wasser getaucht und dann 16 h bei Raumtemperatur in 1,5-normaler Schwefelsäure gelagert. Anschließend wird die Elektrode erneut mit Wasser neutral gewaschen, nachfolgend nochmals 30 min in siedendes Wasser getaucht und dann abgekühlt.

## Patentansprüche

1. Implantierbarer Defibrillator mit einer Flächenelektrode in Form eines Netzes, einer Spirale oder eines Gewebes aus elektronisch leitendem Material, **dadurch gekennzeichnet,** daß die Elektrode vollständig in ein biokompatibles, hydrophiles, elektrolytisch leitendes Polymer eingebettet ist.

2. Implantierbarer Defibrillator nach Anspruch 1, **dadurch gekennzeichnet,** daß die Dicke der Elektrode ≦ 300 µm beträgt.

3. Implantierbarer Defibrillator nach Anspruch 1 oder 2, **dadurch gekennzeichnet,** daß die Elektrode aus leitfähigem Kohlenstoff besteht.

4. Implantierbarer Defibrillator mit einer intrakardialen Elektrode in Form einer Wendel aus elektronisch leitendem Material, **dadurch gekennzeichnet,** daß die Elektrode vollständig von einem biokompatiblen, hydrophilen, elektrolytisch leitenden Polymer umhüllt ist.

5. Implantierbarer Defibrillator nach einem der Ansprüche 1, 2 und 4, **dadurch gekennzeichnet,** daß die Elektrode aus Platin oder Platin/Iridium besteht.

6. Implantierbarer Defibrillator nach einem der Ansprüche 1 bis 5, **dadurch gekennzeichnet,** daß das Polymer ein Kationenaustauscher ist.

7. Implantierbarer Defibrillator nach Anspruch 6, **dadurch gekennzeichnet,** daß der Kationenaustauscher aus Poly(perfluoralkylen)-sulfonsäure besteht.

8. Implantierbarer Defibrillator nach einem der Ansprüche 1 bis 7, **dadurch gekennzeichnet,** daß in das Polymer ein Steroid eingelagert ist.

9. Verfahren zur Herstellung eines implantierbaren Defibrillators nach einem oder mehreren der Ansprüche 1, 2 und 5 bis 7, **dadurch gekennzeichnet,** daß ein Netz oder eine Spirale aus elektronisch leitendem Material zwischen zwei Membranen aus einem biokompatiblen, hydrophilen, elektrolytisch leitenden Polymer angeordnet wird, daß diese Anordnung bei erhöhter Temperatur und erhöhtem Druck gepreßt wird und daß das Netz bzw. die Spirale mit einer Kontaktierung versehen wird.

10. Verfahren zur Herstellung eines implantierbaren Defibrillators nach einem der Ansprüche 3 bis 7, **dadurch gekennzeichnet,** daß ein Gewebe aus leitfähigem Kohlenstoff oder eine Wendel aus elektronisch leitendem Material mit einer Lösung eines biokompatiblen, hydrophilen, elektrolytisch leitenden Polymers behandelt wird und daß das Gewebe bzw. die Wendel mit einer Kontaktierung versehen wird.
